# EUROPÄISCHE PATENTSCHRIFT

(11) **EP 2 649 040 B1**
(45) Veröffentlichungstag und Bekanntmachung des Hinweises auf die Patenterteilung: **12.10.2016**
(21) Anmeldenummer: 11796660.6
(22) Anmeldetag: 07.12.2011
(51) Int. Cl.: C07C 209/16, C07C 213/02, C07C 227/08, C07C 231/12, C07C 319/20, C07D 213/38, C07D 307/52, C07C 211/07, C07C 211/27, C07C 217/08, C07C 229/08, C07C 237/06, C07C 323/25, C07C 229/38

(54) **VERFAHREN ZUR HOMOGEN-KATALYSIERTEN, HOCHSELEKTIVEN DIREKTEN AMINIERUNG VON PRIMÄREN ALKOHOLEN MIT AMMONIAK ZU PRIMÄREN AMINEN BEI HOHEM VOLUMENVERHÄLTNIS VON FLÜSSIG- ZU GASPHASE UND/ODER HOHEN DRÜCKEN**
PROCESS FOR HOMOGENEOUSLY CATALYZED, HIGHLY SELECTIVE DIRECT AMINATION OF PRIMARY ALCOHOLS WITH AMMONIA TO PRIMARY AMINES WITH A HIGH VOLUME RATIO OF LIQUID PHASE TO GAS PHASE AND/OR HIGH PRESSURES
PROCÉDÉ PAR CATALYSE HOMOGÈNE D'AMINATION DIRECTE HAUTEMENT SÉLECTIVE, D'ALCOOLS PRIMAIRES AVEC L'AMMONIAC, POUR DONNER DES AMINES PRIMAIRES, SOUS DE HAUTS RAPPORTS VOLUMIQUES ENTRE LA PHASE LIQUIDE ET LA PHASE GAZEUSE ET/OU EN PRÉSENCE DE PRESSIONS ÉLEVÉES

(30) Priorität: 08.12.2010 DE 102010062645; 21.02.2011 DE 102011004470; 03.05.2011 DE 102011075162
(43) Veröffentlichungstag der Anmeldung: 16.10.2013
(73) Patentinhaber: Evonik Degussa GmbH, 45128 Essen (DE)
(72) Erfinder: KLASOVSKY, Florian, 45721 Haltern am See (DE); PFEFFER, Jan Christoph, 63452 Hanau (DE); TACKE, Thomas, 63755 Alzenau (DE); HAAS, Thomas, 48161 Münster (DE); BELLER, Matthias, 18211 Nienhagen (DE); KÖCKRITZ, Angela, 12437 Berlin (DE); DEUTSCH, Jens, 15834 Rangsdorf (DE); MARTIN, Andreas, 12524 Berlin (DE); IMM, Sebastian, 63505 Langenselbold (DE)
(86) Internationale Anmeldenummer: PCT/EP2011/071992
(87) Internationale Veröffentlichungsnummer: WO 2012/076560

(56) Entgegenhaltungen:
- WO-A1-2010/018570
- US-A- 4 210 605
- IMM, SEBASTIAN ET AL: "An Efficient and General Synthesis of Primary Amines by Ruthenium-Catalyzed Amination of Secondary Alcohols with Ammonia", ANGEWANDTE CHEMIE, INTERNATIONAL EDITION, 49(44), 8126-8129, S8126/1-S8126/9 CODEN: ACIEF5; ISSN: 1433-7851, 2010, XP002669003,

## Beschreibung

Die vorliegende Erfindung betrifft ein chemokatalytisches Flüssigphasen-Verfahren zur hochselektiven, direkten einstufigen Aminierung von primären Alkoholen zu primären Aminen mit Ammoniak mithilfe eines homogenen Katalysatorsystems bei hohem Volumenverhältnis von Flüssig- zu Gasphase (V_{Fl}/V_{Gas}) und/oder hohen Drücken.

### Stand der Technik

Die Umwandlung von sauerstoffhaltigen in stickstoffhaltige funktionelle Gruppen stellt eine essentielle Transformation für die Synthese einer Vielzahl organischer Verbindungen dar. In Literatur und Technik sind hierzu eine Reihe klassischer Methoden bekannt.
In der überwiegenden Mehrzahl der Veröffentlichungen wird ein primärer oder sekundärer Alkohol mit einem primären oder sekundären organischen Amin zur Reaktion gebracht. Die direkte Umsetzung eines primären oder sekundären Alkohols mit Ammoniak zu primären Aminen unten gezeigtem Reaktionsschema ist dagegen nur unter Anwendung besonderer Verfahrensbedingungen, Katalysatoren und nur mit einigen wenigen Alkoholen beschrieben worden.

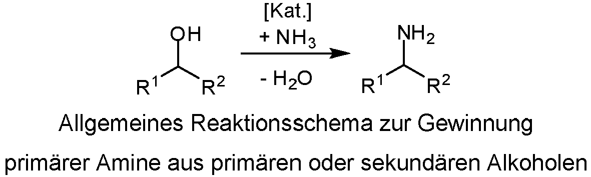

Die Herausforderung aller bekannten Verfahren besteht in der Erzielung hoher Selektivitäten zu den primären Aminen, da diese nucleophiler als Ammoniak sind und infolgedessen bevorzugt unter Bildung höherer Amine reagieren können. Während die Umwandlung einer isolierten Hydroxyl- in eine Aminofunktion annähernd thermoneutral verläuft, ist die Bildung sekundärer und tertiärer Amine mit je ca. 30 kJ/mol exotherm und daher auch thermodynamisch gegenüber der Bildung primärer Amine bevorzugt.

### Direkte Aminierung in der Gasphase

Die einstufige direkte Umwandlung einer primären oder sekundären Hydroxylgruppe unter Beibehaltung der Oxidationsstufe des die Hydroxylgruppe tragenden Kohlenstoffatoms mit Ammoniak in ein primäres Amin ist im Falle niederer, leicht verdampfbarer Alkohole hauptsächlich auf Gasphasenreaktionen beschränkt. Hierbei wird der entsprechende Alkohol verdampft und unter geeigneten Bedingungen (Druck, Temperatur, Wasserstoff- und ggf. Inertgaspartialdruck) an einem vorwiegend heterogenen Katalysator zur Reaktion gebracht. Diese Verfahrensweise wird zum Beispiel in den Publikationen US 4314084, US 5530127, US 5932769, FR 1347648, US 3270059, US 4111840, US 4123462, DE 1667193, Fischer et al. (J. Catal., 1999, 182, 289-291) oder Jenzer et al. (Catal. Lett., 1999, 61, 111-114) beschrieben. Nachteil der meisten heterogenkatalysierten Gasphasenvefahren ist die Anwendung hoher Temperaturen (bis zu 400°C) und Drücke (bis zu 300 bar), in deren Folge neben den gewünschten primären Aminen häufig erhebliche Mengen höherer Amine, Alkene und Alkane entstehen. Entsprechend den charakteristischen Druck- und Temperaturbedingungen einer Gasphasenreaktion können mit den genannten Verfahren zudem lediglich solche Substrate in wirtschaftlichen Ausbeuten zu Aminen umgesetzt werden, die sich verlustfrei verdampfen und umsetzen lassen, bzw. wobei sich die Amine verlustfrei kondensieren bzw. resublimieren lassen. Substrate bzw. ihre korrespondierenden Amine, die unter solchen Bedingungen einer Zersetzung unterliegen, werden in Literatur und Technik daher in Flüssigphasensynthesen umgesetzt.

### Reduktive Aminierung

Dem Fachmann bekannte Verfahren zur Darstellung primärer Amine aus Alkoholen in flüssiger Phase mittels reduktiver Aminierung nutzen eine mehrstufige Vorgehensweise, die mit einem Wechsel des Oxidationszustands des die Hydroxylgruppe tragenden Kohlenstoffatoms einhergehen kann. Unter Wechsel der Oxidationsstufe des die Hydroxylgruppe tragenden Kohlenstoffatoms (reduktive Aminierung) können Alkohole klassischerweise durch Oxidation zur entsprechenden Carbonylverbindung, nachfolgende Bildung des Imins durch Reaktion mit einer Aminkomponente (primäre, sekundäres Amin oder Ammoniak) und die anschließende homogen- bzw. heterogenkatalysierte Reduktion des Imins mit Wasserstoff dargestellt werden. Die zweistufige Vorgehensweise unter Isolierung der Carbonylverbindung ist jedoch zeit- und kostensintensiv.

### Spezielle Mehrstufenprozesse

Unter Beibehaltung der Oxidationsstufe des die Hydroxylgruppe tragenden Kohlenstoffatoms (direkte Aminierung) können Alkohole durch mehrstufige Substitutionsreaktionen zu Aminen umgesetzt werden. Neben dem Aufwand zur Isolierung der Zwischenstufen wirkt sich bei entsprechenden Verfahren insbesondere der Umgang mit den in diesem Zusammenhang häufig angewandten explosiven und giftigen Aziden als nachteilig aus.
Ausnahmen von der mehrstufigen Arbeitsweise zur direkten Aminierung von Alkoholen unter Beibehaltung der Oxidationsstufe des die Hydroxylgruppe tragenden Kohlenstoffatoms stellt beispielsweise die sequentielle Umsetzung primärer Alkohole mit Dialkylazodicarboxylaten, Bis-tert-butyliminodicarbonat und immobilisiertem Triphenylphosphan dar, die gemäß Sun et al. (Tetrahedron Lett., 2007, 48, 7745-7746) nach Zugabe von Trifluoressigsäure den direkten Zugang zum primären Amin ohne vorherige Isolierung von Zwischenstufen gestattet.
Fabiano et al. (Synlett, 1987, 1987, 190-192) setzen zum gleichen Zweck anstelle von Bis-tert-butyliminodicarbonat die giftige Stickstoffwasserstoffsäure ein.

### Direkte Flüssigphasenaminierung von Alkoholen

Die direkte einstufige Flüssigphasenaminierung gegebenenfalls mehrwertiger primärer Alkohole mit Ammoniak ist in der wissenschaftlichen und Patentliteratur schon länger beschrieben. In einigen Fällen können die beschriebenen Verfahren aufgrund der angewandten Prozessbedingungen nicht eindeutig als Gas- oder Flüssigphasenverfahren eingeordnet werden.
Bei Temperaturen um 170 °C und einem Druck von 200 bar kann nach DE 19507007 Ethanolamin an oxidgeträgerten Ruthenium-Katalysatoren zu Ethylendiamin aminiert werden, wobei die erzielbaren Ausbeuten unter 40 % bleiben.
Die Darstellung einwertiger, gegebenenfalls funktionalisierter primärer Amine in hohen Ausbeuten aus den entsprechenden einwertigen, gegebenenfalls funktionalisierten primären Alkoholen werden in den Arbeiten von Gunanathan et al. beschrieben (Angew. Chem. Int. Ed., 2008, 47, 8661-8664). Hierin wird die direkte einstufige Aminierung z.T. mit Heteroatomen substituierter primärer aliphatischer und benzylischer Alkohole durch 12- bis 36-stündige Umsetzung mit überschüssigem Ammoniak in einem Lösungsmittel bei 7.5 bar und 135-180 °C Reaktionstemperatur beschrieben. Als Katalysator wird der luftstabile Acridinyl-basierte Pincer-Komplex Carbonylchlorohydrido-[4,5-(di-i-propylphosphinomethylacridino) ruthenium(II)] eingesetzt. In diesem Verfahren ist jedoch ersichtlich, dass aliphatische primäre Alkohole zunehmend schlechter umgesetzt werden, insbesondere bei fehlenden Heteroatomsubstituenten und/oder steigende Anteilen an linearen Kohlenstoffketten im Molekül. So werden bei Alkoholen, die mindestens einen aliphatischen Alkylrest enthaltend mindestens drei kovalent miteinander verbundenen Kohlenstoffatome und die keine quartären Kohlenstoffatome aufweisen, nur geringe Ausbeuten zwischen 54 und 82 % erzielt. Es ist daher davon auszugehen, dass keines der vorgenannten Edukte in hoher Selektivität zum entsprechenden primären Amin umgesetzt werden kann, da es zu einer erhöhten Imin- bzw. sekundären Aminbildung kommt.
In WO 2010018570 wird darüber hinaus die Anwendung Chinolinyl-basierter Pincer-Liganden mit vergleichbaren Ausbeuten, jedoch ebenso nur an kurzkettigen Substraten beschrieben.

Die direkte einstufige Flüssigphasenaminierung funktionalisierter, gegebenfalls mehrwertiger Alkohole mit Ammoniak wurde an heterogenen und homogenen Katalysatoren beschrieben. Das Ether-Diol Diethylenglycol wurde in DE 3903367 an verschiedenen Zirkondioxidgeträgerten Cu-Co-Ni-Katalysatoren mit flüssigem Ammoniak bei 200 °C in einer 30 bar Wasserstoffatmosphäre aminiert. Als Reaktionsprodukt wurde jedoch in keinem Fall das Ether-Diamin, sondern lediglich die Folgeprodukte Aminoethoxyethanol und Morpholin isoliert.

Eine Abnahme der Selektivität der Bildung primärer Amine mit zunehmender Kettenlänge des Alkohol-Substrats ist für funktionalisierte sekundäre Alkohole in der Literatur bekannt. So beschreiben Imm et al. (S. Imm, S. Bähn, L. Neubert, H. Neumann, M. Beller, Angew. Chem. 2010, 122(44), 8303-6) eine erhebliche Abnahme der Selektivität zum primären Amin von 76 auf 58 %, wenn 4-Phenyl-2-butanol anstelle von 3-Phenyl-2-propanol in Gegenwart von homogenen Ru-Katalysatoren aminiert wird. In analoger Weise kann bei der Aminierung von aliphatischen sekundären Alkoholen für 2-Nonanol eine deutlich geringere Amin-Ausbeute (51.2 %) als im Falle des niederen Homologen 2-Octanol (67.1 %) beobachtet werden (D. Pingen, C. Müller, D. Vogt, Angew. Chem. 2010, 122(44), 8307-10). Es ist daher davon auszugehen, dass höhere und ggf. zusätzlich funktionalisierte Alkohole auf diesem Wege nicht in hohen Ausbeuten zu den entsprechenden Aminen umgesetzt werden können.

In hohen Ausbeuten von bis zu 95,8 % können gemäß DE 1570542 Polyetherdiole wie Polypropylenglycol direkt zu den korrespondierenden Diaminen umgewandelt werden, wenn die Umsetzung bei 240 °C in Gegenwart von Raney-Nickel-Katalysatoren erfolgt. Jedoch ist auch diese Verfahrensweise für die Umsetzung thermolabiler Substrate ungeeignet.

Unter Verwendung eines Co-Cu-Zn-Katalysators gelingt die Darstellung von Polyetheraminen gemäß US 4153581 jedoch bereits bei 140 °C, ist aber zwingend auf die Anwesenheit von Wasserstoff angewiesen.

Die genannten Beispiele verdeutlichen exemplarisch den Bedarf an Verfahren, eine Aktivierung von insbesondere eher linearen, aliphatischen Alkoholen auch ohne den stöchiometrischen Einsatz schwer zugänglicher und giftiger Hilfssubstanzen sowie von Wasserstoff zu erreichen. Ein entscheidender Nachteil aller bislang für die direkte Flüssigphasen-Aminierung geeigneten Verfahren ist zudem, dass für die Gewinnung und die gegebenenfalls erforderliche Isolierung und Aufreinigung der in der Synthesesequenz auftretenden Intermediate zusätzliche zeit- und kostenintensive Arbeitsschritte vollzogen werden müssen.

Nach dem geschilderten Stand der Technik ist kein Prozess bekannt, der die direkte einstufige, wasserstofffreie Flüssigphasen-Aminierung von primären Alkoholen, insbesondere solcher, die mindestens einen aliphatischen Alkylrest enthaltend mindestens drei kovalent miteinander verbundenen Kohlenstoffatome und keine quartären Kohlenstoffatome aufweisen, mit Ammoniak zu primären Aminen bei milden Reaktionsbedingungen in hohen Ausbeuten beschreibt.

Es war daher eine Aufgabe der vorliegenden Erfindung, ein Verfahren zur Herstellung von primären Aminen ausgehend von primären Alkoholen, insbesondere solcher, die mindestens einen aliphatischen Alkylrest enthaltend mindestens drei kovalent miteinander verbundenen Kohlenstoffatome und keine quartären Kohlenstoffatome aufweisen, bereitzustellen, das mindestens eines der genannten Nachteile umgeht und ökonomisch vorteilhaft durchführbar ist.

### Beschreibung der Erfindung

Überraschenderweise wurde nun ein Verfahren gefunden, das die direkte Aminierung von primären Alkoholen, insbesondere solcher, die mindestens einen aliphatischen Alkylrest enthaltend mindestens drei kovalent miteinander verbundenen Kohlenstoffatome und keine quartären Kohlenstoffatome aufweisen, mit Ammoniak in Gegenwart eines homogenen Katalysators in hohen Ausbeuten erlaubt, wobei die primäre Hydroxylgruppe des Alkohols aminiert wird.

Gegenstand der vorliegenden Erfindung ist daher ein Verfahren, welches die direkte, homogen katalysierte Flüssigphasenaminierung von primären Alkoholen, insbesondere solcher, die mindestens einen aliphatischen Alkylrest enthaltend mindestens drei kovalent miteinander verbundenen Kohlenstoffatome und keine quartären Kohlenstoffatome aufweisen, insbesondere mit einer überstöchiometrischen Menge an Ammoniak bezogen auf zu aminierende Hydoxylgruppen, bevorzugt in Abwesenheit von Wasserstoff, gestattet, wobei die Selektivitätssteigerung durch die Anwendung eines hohen Prozessdrucks und/oder, bevorzugt und, eines hohen Volumenverhältnisses von Flüssig- zu Gasphase zustande kommt.

Ein Vorteil des erfindungsgemäßen Verfahrens ist es, dass die bei der Umsetzung ansonsten notwendige Isolierung und Aufreinigung von Zwischenstufen vermieden wird.
Noch ein Vorteil ist es, dass der Einsatz von problematischen Hilfsstoffen wie beispielsweise Aziden vermieden werden kann. Ein zusätzlicher Vorteil ist weiterhin, dass durch das erfindungsgemäße Verfahren die Bildung von Koppelprodukten entfällt und die Bildung von Nebenprodukten durch geschickte Wahl von Prozessbedingungen auf ein niedriges Maß abgesenkt werden kann.

Vorteilhaft ist es weiterhin, dass der Alkohol in gelöstem Zustand zur Reaktion gebracht wird. Noch ein Vorteil ist, dass die Aminierung des Alkohols ohne eine Isolierung und/oder Aufreinigung von Zwischenstufen oder Intermediaten bewerkstelligt werden kann.

Das erfindungsgemäße Verfahren zur Herstellung von primären Aminen umfasst die Verfahrensschritte
A) Bereitstellung einer Lösung eines primären Alkohols in einer fluiden, nicht gasförmigen Phase,
B) in Kontakt Bringen der Phase mit freiem Ammoniak und/oder mindestens einer Ammoniak freisetzenden Verbindung und einem homogenen Katalysator und gegebenenfalls
C) Isolierung des in Verfahrensschritt B) gebildeten primären Amins,
und ist dadurch gekennzeichnet, dass das Volumenverhältnis des Volumens der Flüssigphase zu dem Volumen der Gasphase im Verfahrensschritt B größer 0,05, bevorzugt größer 0,1, insbesondere größer 0,2 ist und/oder
dass Verfahrensschritt B bei Drücken größer als 10 bar, bevorzugt größer 15 bar, insbesondere größer 20 bar durchgeführt wird.

Unter dem Begriff "primäres Amin" werden im Zusammenhang mit der vorliegenden Erfindung ebenfalls dessen Salze sowie Mischungen des Amins und/oder seiner Salze verstanden. Unter dem Begriff "primärer Alkohol" wird im Zusammenhang mit der vorliegenden Erfindung eine organische Verbindung verstanden, die mindestens eine primäre Hydroxygruppe (R-CH₂(OH) mit R = organischer Rest oder H) aufweist.

Unter dem Begriff "quartäres Kohlenstoffatom" wird ein Kohlenstoffatom verstanden, welches jeweils eine kovalente Einfachbindung zu vier Kohlenstoffatomen ausgebildet hat.

Erfindungsgemäß bevorzugte Verfahren sind dadurch gekennzeichnet, dass das Volumenverhältnis des Volumens der Flüssigphase zu dem Volumen der Gasphase im Verfahrensschritt B größer 0,05, bevorzugt größer 0,1, insbesondere größer 0,2 ist und
dass Verfahrensschritt B bei Drücken größer als 10 bar, bevorzugt größer 15 bar, insbesondere größer 20 bar durchgeführt wird; insbesondere beträgt das Volumenverhältnis des Volumens der Flüssigphase zu dem Volumen der Gasphase im Verfahrensschritt B mehr als 0,2 und der Druck in Verfahrensschritt B ist größer als 20 bar.

Es ist erfindungsgemäß bevorzugt, dass der Ammoniak in Verfahrensschritt B) bezogen auf die Hydroxylgruppen im primären Alkohol in einem molaren Verhältnis von mindestens 5 zu 1, bevorzugt 50 zu 1, besonders bevorzugt 500 zu 1, eingesetzt wird. Der Ammoniaküberschuss führt zu einer schneller Umsetzung und höheren Selektivität.

Die in dem erfindungsgemäßen Verfahren eingesetzten Alkohole können neben der primären Hydroxygruppe auch weitere Hydroxygruppen aufweisen, so dass ein Polyol aufweisend mindestens eine primäre Hydroxygruppe ebenfalls ein "primärer Alkohol" im Sinne der vorliegenden Erfindung ist. Die Alkohole können darüber hinaus noch weitere Heteroatome im Molekül aufweisen. Beispiele solcher Alkohole können ausgewählt sein aus der Gruppe der aliphatischen unverzweigen oder verzweigten Alkohole (z.B. Methanol, Ethanol, 1-Propanol, 1-Butanol, 2-Methyl-1-Pentanol,...) oder auch der benzylischen Alkohole (z.B. Benzylalkohol, Furfurylalkohol, Nicotinylalkohol,...).

Das erfindungsgemäße Verfahren ist ebenso vorteilhaft einzusetzen, für primäre Alkohole, die eine Carboxygruppe oder Estergruppe, insbesondere eine Carboxygruppe aufweisen.

Es wurde überraschend festgestellt, dass unter den Reaktionsbedingungen mit diesen Substraten bevorzugt nur an der primären OH-Gruppe eine Modifikation stattfindet, da das Verfahren hoch selektiv ist.

Bevorzugte Carboxygruppen enthaltende primäre Alkohole sind insbesondere omega-HydroxyCarbonsäuren, insbesondere abstammend von Fettsäuren. Beispielhafte Quellen solcher Fettsäuren können die Schnitte von Kokosfett, Kernölen oder Ricinusöl sein. Beispiele solcher Carboxygruppen enthaltender primärer Alkohole können ausgewählt sein aus der Gruppe 6-Hydroxyhexansäure, 11-Hydroxyundecansäure oder 12-Hydroxydodecansäure.
Bevorzugte Estergruppen enthaltende primäre Alkohole sind insbesondere ausgewählt aus der Gruppe der Methylester, Ethylester, n-Propylester und iso-Propylester der omega-Hydroxycarbonsäuren, insebesondere ausgewählt aus der Gruppe des 6-Hydroxyhexansäuremethylesters, des 11-Hydroxyundecansäuremethylesters oder des 12-Hydroxydodecansäuremethylesters.

In dem erfindungsgemäßen Verfahren weiterhin bevorzugt einzusetzende Alkohole weisen mindestens einen aliphatischen Alkylrest enthaltend mindestens drei kovalent miteinander verbundenen Kohlenstoffatome und kein quartäres Kohlenstoffatom auf.
Insbesondere enthalten die Alkohole bevorzugt keine Heteroatome. Insbesondere sind in dem erfindungsgemäßen Verfahren auch Alkohole einzusetzen, die dadurch gekennzeichnet sind, dass der aliphatische Alkylrest ein linearer oder verzweigter Alkylrest, enthaltend mindestens 4, bevorzugt mindestens 6, insbesondere mindestens 9 Kohlenstoffatome, ist. Besonders bevorzugt einzusetzenden Alkohole umfassen 1-Butanol, 2-Methyl-1-propanol, 1-Pentanol, 3-Methyl-1-butanol, 2-Methyl-1-butanol, 1-Hexanol, 4-Methyl-1-pentanol, 3-Methyl-1-pentanol, 2-Methyl-1-pentanol, 2-Ethyl-1-butanol, Tripropylenglycol und die Anhydrohexitole, insbesondere umfassend Tripropylenglycol.

Beispielhafte im erfindungsgemäßen Verfahren eingesetzten Alkohol-Konzentrationen bewegen sich im Bereich zwischen 0,1 und 10000 mmol/L, bevorzugt zwischen 0,1 und 1000 mmol/L und besonders bevorzugt zwischen 0,1 und 100 mmol/L, bezogen auf die fluide Phase.

Als in dem erfindungsgemäßen Verfahren einzusetzenden homogenen Katalysatoren kommen alle solche, dem Fachmann bekannten homogenen Katalysatoren in Betracht, die in der Lage sind, die CH-Bindung des die zu aminierende OH-Gruppe tragenden Kohlenstoffatoms zu aktivieren. Beispiele solcher Katalysatoren umfassen die Alkalimetall-, Aluminium- und Lanthanid-Alkoxide, anorganische Verbindungen von Edelmetallen (z. B. [RuCl₃ * nH₂O], IrC1₃), mono- oder multimetallische, ein- oder mehrkernige Koordinationsverbindungen von einem oder mehreren Edelmetallen ausgewählt aus den Elementen Ruthenium (z.B. [RuCl₂(PPh₃)₃], [RuH₂(PPh₃)₄], der Shvo-Katalysator ([(η⁴-C₄Ph₄CO)Ru(CO)₃]₂), [Ru(cod)(cot)], [(PPh₃)₂Ru(CH₃CN)₃Cl]BPh₄, [Ru(p-cymene)Cl₂]₂, [Ru(p-cymene)Cl₂]₂/DPEphos, [Ru(PPh₃)₃(CO)H_{2]}, [Ru₃(CO)₁₂], [Ru₃(CO)₁₂]/N-phenyl-2-(PCl₂)pyrrol, [RuCl₂(dmso)₄]), Rhodium (z. B. der Wilkinson-Katalysator ([RhCl(PPh₃)₃]), [RhH(PPh₃)₃]), Iridium (z. B. [lrCl₃(dmso)₃], [Cp*IrCl₂]₂, [Ir(cod)Cl]₂/(dppp)/Cs₂CO₃, [IrCl₂H(cod)]₂, KOH-aktivierte Phenanthrolin-Iridium-Komplexe) und Palladium ([Pd(PPh₃)₄], [PdCl₂(dppe)], [Pd(OAc)₂]) sowie der anderen Platinmetalle und Eisen.
Erfindungsgemäß bevorzugte Verfahren setzen als homogene Katalysatoren Verbindungen ein, ausgewählt aus der Gruppe bestehend aus Carbonylchlorohydrido-[4,5-(di-i-propylphosphinomethylacridino) ruthenium(II)], Carbonylchlorohydrido-[2-(Dicyclohexylphosphino)-1-phenyl-1 H-pyrryl) ruthenium(II)], Carbonylchlorohydrido-[2-(Di-1-adamantylphosphino)-1-phenyl-1H-pyrryl) ruthenium(II)], Carbonylchlorohydrido-[2-(Diisopropylphosphino)-1-phenyl-1 H-pyrryl) ruthenium(II)], sowie Rutheniumverbindungen mit Liganden des CataCXium® P-Typs.

In einer weiteren bevorzugten Ausführungsform des erfindungsgemäßen Verfahrens werden in Schritt B) Katalysatoren eingesetzt, die dem Fachmann als Katalysatoren zur Hydroformylierung bekannt sind. Hierfür können Übergangsmetallcarbonylverbindungen der allgemeinen Form HₓM_{y}M'_{y'}(CO)_{z}Lₙ eingesetzt werden, wobei n=0 ("unmodifizierte Hydroformylierungskatalysatoren") bzw. n≠0 ("modifizierte Hydroformylierungskatalysatoren") sein kann und im übrigen x, y und z ganzzahlige ganze Werte annehmen. y' kann null sein, wenn ein monometallischer Katalysator eingesetzt wird, oder y' kann einen positiven ganzzahligen Wert annehmen, wenn ein bimetallischer Katalysator verwendet wird. M und M' können gleich oder verschieden sein. Als Übergangsmetalle M und M' können Rhodium, Kobalt, Iridium, Ruthenium, Osmium, Platin, Palladium, Eisen, Nickel, Chrom, Molybdän oder Mangan eingesetzt werden; bevorzugt werden Rhodium, Kobalt, Iridium, Ruthenium, Osmium oder Platin verwendet. Der Ligand L kann ausgewählt sein aus der Gruppe der Phosphane, Phosphanoxide, Phosphite, Amine, Amide, Isonitrile, Arsane oder Stibane; beispielhafte Vertreter sind Triphenylphosphan, Triphenylphosphanoxid, Triphenylphosphantrisulfonsäure Natriumsalz, Triphenylamin oder Triphenylarsan. Beispielhafte Hydroformylierungskatalysatoren sind ausgewählt aus der Gruppe umfassend HCo(CO)₄, HCo(CO)₃PBu₃, HRh(CO)(PR₃)₃, Rh₄(CO)₁₂, Rh₆(CO)₁₆, Rh₂(CO)₄Cl₂, CoRh(CO)₇, Co₂Rh₂(CO)₁₂, HRh(CO)₃.

In diesem Zusammenhang bevorzugter Hydroformylierungskatalysator ist ein Katalysatorsystem enthaltend mindestens einen Xantphos-Liganden der allgemeinen Formel 1 und eine Übergangsmetallverbindung eingesetzt.
Begriff "Xantphos-Ligand" wird im Zusammenhang mit der vorliegenden Erfindung eine Verbindung der allgemeinen Formel 1 verstanden, 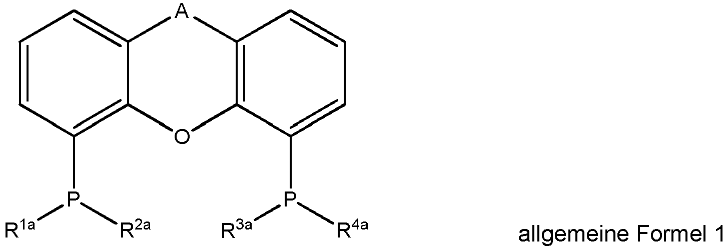 wobei
Erz, R^{2a} , R^{3a} und R^{4a} unabhängig voneinander gleich oder verschieden ausgewählt aus der Gruppe enthaltend, bevorzugt bestehend aus, Phenyl, tert. Butyl und Isopropyl, und
A ausgewählt aus der Gruppe enthaltend, bevorzugt bestehend aus, -C(CH₃)₂-,-CH₂CH₂-,-Si(CH₃)₂-, -S-, -O-, -C(C(CH₃)₂)- und 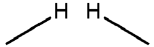
Bevorzugt werden Xantphos-Linganden eingesetzt, bei denen R¹ = R² = R³ = R⁴ = Phenyl und A = -C(CH₃)₂-.
In einer bevorzugt alternativen Ausführungsform werden Xantphos-Linganden eingesetzt, bei denen R¹ = R² = R³ = R⁴ = Phenyl und A = 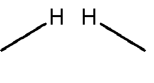 (auch bekannt als DPEphos).

Das Übergangsmetall ist bevorzugt ausgewählt aus der Gruppe enthaltend, bevorzugt bestehend aus, Ruthenium, Kobalt, Rhodium, Iridium, Nickel, Palladium und Platin sowie der anderen Platinmetalle und Eisen. Besonders bevorzugt ist das Übergangsmetall ausgewählt aus der Gruppe bestehend aus Ruthenium, Iridium und Palladium; besonders bevorzugt aus der Gruppe bestehend aus Ruthenium und Iridium, insbesondere Ruthenium.

Es sei erwähnt, dass in Abhängigkeit von der gewählten Kombination aus den beschriebenen, den Katalysator bildenden Elementen dieser eine elektrische Ladung aufweisen und in Form eines mithilfe entsprechender Gegenionen gebildeten Salzes eingesetzt werden kann.

In einer besonders bevorzugten Ausführungsform ist der Katalysator die Xanthen-basierte Koordinationsverbindung Carbonylchlorohydrido-[9,9-Dimethyl-4,5-bis(diphenylphosphino)-xantheno] ruthenium(II)]:

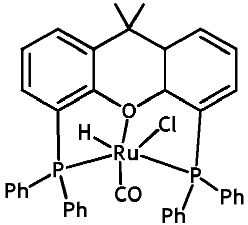

Carbonylchlorohydrido-[9,9-Dimethyl-4,5-bis(diphenylphosphino)xantheno] ruthenium(II)

Es sei erwähnt, dass in Abhängigkeit von der gewählten Kombination aus den beschriebenen, den Katalysator bildenden Elementen dieser eine elektrische Ladung aufweisen und in Form eines mithilfe entsprechender Gegenionen gebildeten Salzes eingesetzt werden kann.

Die im Verfahrensschritt A) eingesetzte fluide Phase kann von einem Lösemittel oder einem unter den Prozessbedingungen in verflüssigter oder überkritischer Form vorliegenden Gas, insbesondere Ammoniak, bzw. Mischungen aus den genannten Komponenten gebildet werden.

Als Lösungsmittel können in diesem Zusammenhang Wasser, oder organische Lösungsmittel oder Mischungen derselben eingesetzt werden, diese Mischungen können eine homogene Lösung oder aber auch eine Emulsion darstellen. Besonders bevorzugt ist der Einsatz mindestens eines organischen Lösungsmittels. Eine nicht als Limitierung anzusehende Auswahl geeigneter organischer Lösmittel umfasst Benzol, Toluol, die Xylol-Isomere, Mesitylen, Dioxan, THF, Dimethoxyethan, Anisol, Cyclohexan und tert.-Butylalkohol.

Als im Verfahrensschritt B) eingesetzter Ammoniak oder eine Ammoniak freisetzende Verbindungen wird im Zusammenhang mit der vorliegenden Erfindung insbesondere auch flüssiger oder überkritischer Ammoniak und/oder eine Lösung von Ammoniumsalzen in einem Lösungsmittel (wie z.B. auch Ammoniumhydroxid in Wasser) verstanden.
Bevorzugt wird in Verfahrensschritt B) als freier Ammoniak gasförmiger oder verflüssigter Ammoniak eingesetzt.

Verfahrensschritt B) wird bei Überdruck, bezogen auf Atmosphärendruck, durchgeführt. Beispielhafte Drücke im Verfahrensschritt B) des erfindungsgemäßen Verfahrens liegen im Bereich zwischen 20 und 1000 bar, bevorzugt zwischen 20 und 500 bar, und besonders bevorzugt zwischen 20 und 100 bar. Der Druck kann durch Einpressen des Ammoniaks und/oder eines weiteren Gases, insbesondere eines Inertgases wie beispielsweise Stickstoff oder Argon, aufgebaut werden, wobei der Druckaufbau durch Gasmischungen der beiden bevorzugt ist.

Die das erfindungsgemäße Verfahren beschreibenden Temperaturen in Verfahrensschritt B) bewegen sich in einem solchen Bereich, der die aufgrund thermischer Belastung zur Bildung von Nebenprodukten führenden Zersetzungsreaktionen von primärem Alkohol, primärem Amin und allen weiteren im Zuge des Verfahrens auftretenden Intermediaten auf ein Minimum beschränkt. Beispielhaft bewegen sich die Temperaturen in einem Bereich zwischen 80 und 220 °C, bevorzugt zwischen 90 und 200 °C und besonders bevorzugt zwischen 100 und 170 °C, gemessen in der fluiden Phase.

Es ist erfindungsgemäß bevorzugt, dass das Verfahren in Abwesenheit von Wasserstoff durchgeführt wird, wobei unter Abwesenheit von Wasserstoff verstanden wird, dass kein Wasserstoff zusätzlich zur Reaktion zugeführt wird; gegebenenfalls in der Luft enthaltene Spuren von Wasserstoff gelten als "in Abwesenheit von Wasserstoff" im Sinne der vorliegenden Erfindung.

### Beispiele

### Beispiel 1: Direkte einstufige Aminierung von 1-Hexanol mit Ammoniak an einem homogenen Rutheniumkatalysator bei niedrigem V_{Fl}/V_{Gas} Vergleichsbeispiel, nicht erfindungsgemäß V_{Fl}/V_{Gas} = 0,05).

Unter Argon-Atmosphäre wurden 1,022 g (10 mmol) 1-Hexanol, 0,006 g (0,01 mmol) Carbonylchlorohydrido-[4,5-(di-i-propylphosphinomethylacridino) ruthenium(II)] als Katalysator, 1 mL Wasser und 2 mL Dioxan als Lösemittel in einen 90 mL Fischer-Porter-Reaktor gegeben. Der Behälter wurde verschlossen, dreimal je 20 bar Argon aufgepresst und entspannt. Danach wurde Ammoniak bis zu einem Druck von 7,5 bar in den Autoklaven eingefüllt (insgesamt gilt V_{Fl}/V_{Gas} = 0,05), der Reaktor auf 135 °C aufgeheizt und 30 h bei dieser Temperatur gehalten. Nach Abkühlen auf Raumtemperatur wurde der Reaktor geöffnet und das Reaktionsprodukt gaschromatographisch analysiert. Es wird 1-Hexylamin mit einer Ausbeute von 79,7 % erhalten (99 % Umsatz).

### Beispiel 2: Direkte einstufige Aminierung von Furfurylalkohol mit Ammoniak an einem homogenen Rutheniumkatalysator bei niedrigem V_{Fl}/V_{Gas} Vergleichsbeispiel, nicht erfindungsgemäß V_{Fl}/V_{Gas} = 0.05).

Unter Argon-Atmosphäre wurden 0,098 g (1 mmol) Furfurylalkohol, 0,0128 g (0,02 mmol) Trirutheniumdodecacarbonyl und 0,0204 g (0,06 mmol) 2-(Decyclohexylphosphanyl)-1-phenyl-1-H-pyrrol als Katalysator sowie 1 mL 2-Methyl-2-butanol als Lösemittel in ein 50 mL Stahlrohr gegeben. Der Behälter wurde verschlossen, dreimal je 20 bar Argon aufgepresst und entspannt. Danach wurde der Behälter mit Trockeneis gekühlt und 0,6 g (1 mL, 35.3 mmol) Ammoniak einkondensiert (ingesamt gilt V_{Fl}/V_{Gas} = 0,05), der Reaktor auf 150 °C aufgeheizt und 20 h bei dieser Temperatur gehalten. Nach Abkühlen auf Raumtemperatur wurde der Reaktor geöffnet, das Lösemittel am Rotationsverdampfer entfernt und der Rückstand nach Lösen in Methanol gaschromatographisch analysiert. Es wird Furfurylamin mit einer Ausbeute von 71 % erhalten (99 % Umsatz).

### Beispiel 3: Direkte einstufige Aminierung von Tripropylenglycol mit Ammoniak an einem homogenen Rutheniumkatalysator bei hohem Druck und großem V_{Fl}V_{Gas} erfindungsgemäß V_{Fl}/V_{Gas} = 0.3)

Unter einer Argon-Atmosphäre wurden 0,961 g (5 mmol) Tripropylenglycol, 0,0305 g (0,05 mmol) Carbonylchlorohydrido-[4,5-(di-i-propylphosphinomethylacridino) ruthenium(II)] als Katalysator und 25 ml 2-Methyl-2-butanol als Lösungsmittel in den Glaseinsatz eines 100 ml Hastelloy-Autoklaven gegeben. Der Autoklav wurde verschlossen, dreimal je 20 bar Argon aufgepresst und entspannt und erneut 15 bar Argon aufgepresst. Danach wurden 2 g (2.95 mL; 117 mmol) flüssiger Ammoniak in den Autoklaven eingefüllt (ingesamt gilt V_{Fl}/V_{Gas} = 0,3). Die Reaktionsmischung wurde 10 Minuten bei Raumtemperatur gerührt (600 rpm), anschließend unter Rühren auf 170°C Innentemperatur erhitzt und 48 Stunden bei dieser Temperatur gehalten, wobei sich ein Druck von 45 bar einstellte. Nach Abkühlung auf Raumtemperatur, vorsichtigem Entspannen des Ansatzes und dreimaligem Aufpressen von 20 bar Argon mit nachfolgendem Entspannen wurde der Autoklav geöffnet, das Reaktionsgemisch durch Kieselgur filtriert und das Filtrat zur Entfernung des Lösungsmittels im Vakuum am Rotationsverdampfer eingeengt. Das erhaltene Rohprodukt wurde durch Kugelrohrdestillation im Vakuum gereinigt. Man erhält das Diamin des Tripropylenglycols mit einer Ausbeute von 91 % der Theorie, Siedebereich 90-95°C Luftbadtemperatur bei 10 mbar).

### Beispiel 4: Direkte einstufige Aminierung von 1-Hexanol (Alkohol) mit Ammoniak an einem homogenen Rutheniumkatalysator (Variation Druck und V_{Fl}/V_{Gas}).

Unter Argon-Atmosphäre wurden ***m_{H}** g* 1-Hexanol, ***m_{Ru}** g* [Carbonylchlorohydrido-tris(triphenylphosphan)ruthenium(II)] und ***m_{P}*** g 9,9-Dimethyl-4,5-bis(diphenylphosphino)xanthen als Katalysator sowie ***V_{LM}*** mL 2-Methyl-2-butanol als Lösemittel in ein 50 mL Stahlrohr gegeben. Der Behälter wurde verschlossen, dreimal je 20 bar Argon aufgepresst und entspannt. Danach wurde der Behälter mit Trockeneis gekühlt und ***m_{A}*** g Ammoniak einkondensiert. Nach Aufpressen eines Differenzdrucks von weiteren ***p*** bar Argon wurde der Reaktor auf 130 °C aufgeheizt und 20 h bei dieser Temperatur gehalten. Nach Abkühlen auf Raumtemperatur wird der Reaktor entspannt und geöffnet, das Lösemittel am Rotationsverdampfer entfernt und der Rückstand nach Lösen in Methanol gaschromatographisch analysiert. Reaktionsparameter sowie Umsätze und Selektivitäten zum gewünschten Reaktionsprodukt 1-Hexylamin sind in der folgenden Tabelle wiedergegeben. Die Ergebnisse zeigen, dass die Selektivität zum Zielprodukt sowohl durch Erhöhung des Verhältnisses V_{Fl}/_{Gas} als auch durch Erhöhung des Drucks sowie durch gleichzeitige Erhöhung beider Parameter gesteigert werden kann.

| Nr. | erfindungsgemäß | m_{H} [g]¹ | m_{Ru} [g]² | m_{P} [g]³ | V_{LM} [mL]⁴ | m_{A} [g]⁵ | p [bar]⁶ | V_{FI}/V_{Gas} [-]⁷ | U [%]8 | S [%]⁹ |
|---|---|---|---|---|---|---|---|---|---|---|
| 4.1 | - | 0,10 | 0,029 | 0,017 | 1 | 0,3 | 0 | 0,03 | 100 | 31 |
| 4.2 | + | 0,10 | 0,029 | 0,017 | 1 | 0,3 | 20 | 0,03 | 100 | 37 |
| 4.3 | + | 0,41 | 0,116 | 0,069 | 4 | 1,2 | 0 | 0,14 | 80 | 50 |
| 4.4 | + | 0,41 | 0,116 | 0,069 | 4 | 1,2 | 20 | 0,14 | 65 | 48 |

| | | | | | | | | | | |
|---|---|---|---|---|---|---|---|---|---|---|
| 1: Masse 1-Hexanol; 2: Masse [Carbonylchlorohydridotris-(triphenylphosphan)rutrienium(II)]; 3: Masse Xantphos; 4: Volumen Lösungsmittel; 5: Masse Ammoniak; 6: sich einstellender Druck unter Reaktionsbedingungen; 7: Verhältnis des Flüssigphasenvolumens zum Gasphasenvolumen; 8: Umsatz 1-Hexanol; 9: Selektivität zum 1-Hexylamin. | | | | | | | | | | |

### Beispiel 5: Direkte einstufige Aminierung von 12-Hydroxydodecansäuremethylester (Hydroxysäure) mit Ammoniak an einem homogenen Rutheniumkatalysator (Variation Druck und V_{FI}/V_{Gas}).

Unter Argon-Atmosphäre wurden ***m_{H}** g* 12-Hydroxydodecansäuremethylester, ***m_{Ru}*** g [Carbonylchlorohydrido-tris(triphenylphosphan)ruthenium(II)] und ***m_{P}*** g 9,9-Dimethyl-4,5-bis(diphenylphosphino)xanthen als Katalysator sowie ***V_{LM}*** mL 2-Methyl-2-butanol als Lösemittel in ein 50 mL Stahlrohr gegeben. Der Behälter wurde verschlossen, dreimal je 20 bar Argon aufgepresst und entspannt. Danach wurde der Behälter mit Trockeneis gekühlt und ***m_{A}*** g Ammoniak einkondensiert. Nach Aufpressen eines Differenzdrucks von weiteren ***p*** bar Argon wurde der Reaktor auf 130 °C aufgeheizt und 20 h bei dieser Temperatur gehalten. Nach Abkühlen auf Raumtemperatur wurde der Reaktor entspannt und geöffnet, das Lösemittel am Rotationsverdampfer entfernt und der Rückstand nach Lösen in Methanol gaschromatographisch analysiert. Reaktionsparameter sowie Umsätze und Selektivitäten zum gewünschten Reaktionsprodukt 12-Aminododecansäuremethylester sind in der folgenden Tabelle wiedergegeben. Die Ergebnisse zeigen, dass die Selektivität zum Zielprodukt sowohl durch Erhöhung des Verhältnisses V_{Fl}/V_{Gas} als auch durch Erhöhung des Drucks sowie durch gleichzeitige Erhöhung beider Parameter gesteigert werden kann.

| Nr. | erfindungsgemäß | m_{H} [g]¹ | m_{Ru} [g]² | m_{P} [g]³ | V_{LM} [mL]⁴ | m_{A} [g]⁵ | p [bar]⁶ | V_{FI}/V_{Gas} [-]⁷ | U [%]⁸ | S [%]⁹ |
|---|---|---|---|---|---|---|---|---|---|---|
| 5.1 | - | 0,23 | 0,029 | 0,017 | 1 | 0,3 | 0 | 0,04 | 100 | 30 |
| 5.2 | + | 0,23 | 0,029 | 0,017 | 1 | 0,3 | 20 | 0,04 | 98 | 42 |
| 5.3 | + | 0,92 | 0,116 | 0,069 | 4 | 1,2 | 0 | 0,16 | 96 | 50 |
| 5.4 | + | 0,92 | 0,116 | 0,069 | 4 | 1,2 | 20 | 0,16 | 77 | 61 |

| | | | | | | | | | | |
|---|---|---|---|---|---|---|---|---|---|---|
| 1: Masse 12-Hydroxydodecansäuremethylester; 2: Masse [Carbonylchlorohydridotris-(triphenylphosphan)ruthenium(II)]; 3: Masse Xantphos; 4: Volumen Lösungsmittel; 5: Masse Ammoniak; 6: sich einstellender Druck unter Reaktionsbedingungen; 7: Verhältnis des Flüssigphasenvolumens zum Gasphasenvolumen; 8: Umsatz 12-Hydroxydodecansäuremethylester; 9: Selektivität zur 12-Aminododecansäuremethylester. | | | | | | | | | | |

### Beispiel 6: Direkte einstufige Aminierung von Alkoholen und Hydroxysäuren mit Ammoniak an einem homogenen Rutheniumkatalysator und Xantphos bei hohem V_{Fl}/V_{Gas} (erfindungsgemäß)

Unter Argon-Atmosphäre wurden ***m_{E}*** g Edukt, ***m_{Ru}*** g [Carbonylchlorohydrido-tris(triphenylphosphan)ruthenium(II)] und ***m_{P}*** g 9,9-Dimethyl-4,5-bis(diphenylphosphino)xanthen als Katalysator sowie ***V_{LM}*** mL 2-Methyl-2-butanol als Lösemittel in ein 50 mL Stahlrohr gegeben. Der Behälter wurde verschlossen, dreimal je 20 bar Argon aufgepresst und entspannt. Danach wurde der Behälter mit Trockeneis gekühlt und ***m_{A}*** g Ammoniak einkondensiert. Der Reaktor wurde auf ***T*** °C aufgeheizt und 20 h bei dieser Temperatur gehalten. Nach Abkühlen auf Raumtemperatur wurde der Reaktor entspannt und geöffnet, das Lösemittel am Rotationsverdampfer entfernt und der Rückstand nach Lösen in Methanol gaschromatographisch analysiert. Reaktionsparameter sowie Umsätze und Selektivitäten zum gewünschten Reaktionsprodukten sind in der folgenden Tabelle wiedergegeben. Die Ergebnisse zeigen, dass eine Vielzahl unterschiedlicher hydroxyfunktionalisierter Substrate mit der beschriebenen Methode aminiert werden kann.

| Edukt | m_{E} [g]¹ | m_{Ru} [g]² | m_{P} [g]³ | V_{LM} [mL]⁴ | m_{A} [g]⁵ | T [°C]⁶ | V_{Fl}/V_{Gas} [-]⁷ | U [%]⁸ | S [%]⁹ |
|---|---|---|---|---|---|---|---|---|---|
| Tetraethylenglycol | 0,19 | 0,029 | 0,017 | 1 | 1 | 140 | 0,06 | 100 | 97 |
| p-Hydroxymethylbenzylalkohol | 0,14 | 0,029 | 0,017 | 3 | 1 | 150 | 0,10 | 100 | 48 |
| p-Hydroxymethylbenzylalkohol | 0,14 | 0,029 | 0,017 | 5 | 1 | 150 | 0,15 | 100 | 76 |
| m-Hydroxymethylbenzylalkohol | 0,14 | 0,029 | 0,017 | 5 | 1 | 150 | 0,15 | 100 | 70 |
| 1-Octanol | 0,13 | 0,029 | 0,017 | 1 | 1 | 130 | 0,06 | 99 | 53 |
| 1-Octanol | 0,13 | 0,029 | 0,017 | 3 | 1 | 130 | 0,10 | 80 | 79 |
| 1-Octanol | 0,13 | 0,029 | 0,017 | 3 | 1 | 140 | 0,10 | 99 | 80 |
| 2-Phenylethanol | 0,12 | 0,029 | 0,017 | 3 | 1 | 140 | 0,10 | 99 | 94 |
| Benzylalkohol | 0,11 | 0,029 | 0,017 | 3 | 1 | 140 | 0,10 | 100 | 87 |
| 3-Pyridinylmethanol | 0,11 | 0,029 | 0,017 | 3 | 1 | 140 | 0,10 | 100 | 96 |
| 10-Hydroxydecansäure-methylester | 0,20 | 0,029 | 0,017 | 3 | 1 | 130 | 0,10 | 100 | 75 |
| 4-Hydroxymethylbenzoesäuremethylester | 0,17 | 0,029 | 0,017 | 3 | 0,6 | 130 | 0,09 | 100 | 92 |

| | | | | | | | | | |
|---|---|---|---|---|---|---|---|---|---|
| *1: Masse Edukt;* *2: Masse [Carbonylchlorohydridotris-(triphenylphosphan)ruthenium(II)];* *3: Masse Xantphos;* *4: Volumen Lösungsmittel;* *5 :Masse Ammoniak;* *6: Reaktionstemperatur;* *7: Verhältnis des Flüssigphasenvolumens zum Gasphasenvolumen;* *8: Umsatz Edukt;* *9: Selektivität zum Produkt.* | | | | | | | | | |

### Beispiel 7: Direkte einstufige Aminierung von Glycolsäure und Glycolsäureanilid mit Ammoniak an einem homogenen Rutheniumkatalysator und Xantphos (Variation V_{Fl}/V_{Gas})

Unter Argon-Atmosphäre wurden ***m_{E}*** g Edukt, ***m_{Ru}*** g [Carbonylchlorohydrido-tris(triphenylphosphan)ruthenium(II)] und ***m_{P}*** g 9,9-Dimethyl-4,5-bis(diphenylphosphino)xanthen als Katalysator sowie ***V_{LM}*** mL 2-Methyl-2-butanol als Lösemittel in ein 50 mL Stahlrohr gegeben. Der Behälter wurde verschlossen, dreimal je 20 bar Argon aufgepresst und entspannt. Danach wurde der Behälter mit Trockeneis gekühlt und ***m_{A}*** g Ammoniak einkondensiert. Der Reaktor wurde auf 140 °C aufgeheizt und 20 h bei dieser Temperatur gehalten. Nach Abkühlen auf Raumtemperatur wurde der Reaktor entspannt und geöffnet, das Lösemittel am Rotationsverdampfer entfernt und der Rückstand nach Lösen in Methanol gaschromatographisch analysiert. Reaktionsparameter sowie Umsätze und Selektivitäten zum gewünschten Reaktionsprodukten sind in der folgenden Tabelle wiedergegeben. Die Ergebnisse zeigen, dass bei beiden Substraten durch Erhöhung des Verhältnisses V_{fl}/V_{gas} eine Erhöhung der Selektivität zum gewünschten Produkt erzielt werden kann.

| Edukt | m_{E} [g]¹ | m_{Ru} [g]² | m_{P} [g]³ | V_{LM} [mL]⁴ | m_{A} [g]⁵ | V_{Fl}/V_{Gas} [-]⁶ | U [%]⁷ | S [%]⁸ | |
|---|---|---|---|---|---|---|---|---|---|
| Glycolsäure | 0,076 | 0,029 | 0,017 | 1 | 0,3 | 0,03 | 80 | 10 | |
| Glycolsäure | 0,304 | 0,116 | 0,069 | 4 | 1,2 | 0,14 | 74 | 24 | |
| Glycolsäureanilid | 0,151 | 0,029 | 0,017 | 1 | 0,3 | 0,03 | 55 | 29 | |
| Glycolsäureanilid | 0,605 | 0,116 | 0,069 | 4 | 1,2 | 0,14 | 47 | 38 | |

| | | | | | | | | | |
|---|---|---|---|---|---|---|---|---|---|
| *1: Masse Edukt;* *2: Masse [Carbonylchlorohydridotris-(triphenylphosphan)ruthenium(II)];* *3: Masse Xantphos;* *4: Volumen Lösungsmittel;* *5: Masse Ammoniak;* *6: Verhältnis des Flüssigphasenvolumens zum Gasphasenvolumen;* *7: Umsatz Edukt;* *8: Selektivität zum primären Aminoglycolsäure(-derivat).* | | | | | | | | | |

### Beispiel 8: Direkte einstufige Aminierung von 2-Methylthioethanol mit Ammoniak an einem homogenen Rutheniumkatalysator und Xantphos (Variation V_{Fl}/V_{Gas})

Unter einer Argon-Atmosphäre werden ***m_{M}*** g 2-Methylthioethanol, ***m_{Ru}*** g [Carbonylchlorohydridotris-(triphenylphosphan)ruthenium(II)] als Katalysator, ***m_{P}*** g Xantphos und ***V_{LM}*** ml 2-Methyl-2-butanol als Lösungsmittel in den Glaseinsatz eines 314 ml Hastelloy-Autoklaven gegeben. Der Autoklav wird verschlossen, 5 bar Stickstoff aufgepresst, entspannt und auf -70 °C abgekühlt. Danach werden ***m_{A}*** g flüssiger Ammoniak in den Autoklaven einkondensiert. Die Reaktionsmischung wird anschließend 10 Minuten bei Raumtemperatur gerührt (600 rpm), dann unter Rühren auf 170°C Innentemperatur erhitzt und 48 Stunden bei dieser Temperatur gehalten. Nach Abkühlung auf Raumtemperatur, vorsichtigem Entspannen des Ansatzes sowie Aufpressen von 5 bar Stickstoff mit nachfolgendem Entspannen wird der Autoklav geöffnet und die Reaktionsmischung mithilfe eines Gaschromatographen analysiert. Reaktionsparameter sowie Umsätze und Selektivitäten zum gewünschten primären Amin 2-Methylthioethylamin sind in der nachfolgenden Tabelle wiedergegeben. Die Ergebnisse zeigen, dass die Selektivität zum Zielprodukt durch Erhöhung des Verhältnisses V_{Fl}/V_{Gas} gesteigert werden kann.

| Nr. | m_{O} [g]¹ | m_{Ru} [g]² | m_{P} [g]³ | V_{LM} [mL]⁴ | m_{A} [g]⁵ | V_{Fl}/V_{Gas} [-]⁶ | U[%]⁷ | S[%]⁸ |
|---|---|---|---|---|---|---|---|---|
| 1 | 2,3 | 0,7 | 0,42 | 24,6 | 9,8 | 0,14 | 89 | 42 |
| 2 | 11,7 | 3,57 | 2,18 | 125,2 | 37,5 | 1,68 | 78 | 43 |

| | | | | | | | | |
|---|---|---|---|---|---|---|---|---|
| *1: Masse 2-Methylthioethanol;* *2: Masse [Carbonylchlorohydridotris-(triphenylphosphan)ruthenium(II)];* *3: Masse Xantphos;* *4: Volumen Lösungsmittel;* *5: Masse Ammoniak;* *6: Verhältnis des Flüssigphasenvolumens zum Gasphasenvolumen;* *7: Umsatz 2-Methylthioethanol;* *8: Selektivität zum 2-Methylthioethylamin.* | | | | | | | | |

## Patentansprüche

1. Verfahren zur Herstellung von primären Aminen umfassend die Verfahrensschritte
A) Bereitstellung einer Lösung eines primären Alkohols in einer fluiden, nicht gasförmigen Phase,
B) in Kontakt Bringen der Phase mit freiem Ammoniak und/oder mindestens einer Ammoniak freisetzenden Verbindung und einem homogenen Katalysator und gegebenenfalls
C) Isolierung des in Verfahrensschritt B) gebildeten primären Amins,
**dadurch gekennzeichnet,**
**dass** das Volumenverhältnis des Volumens der Flüssigphase zu dem Volumen der Gasphase im Verfahrensschritt B größer 0,05 ist und/oder
**dass** Verfahrensschritt B bei Drücken größer als 10 bar durchgeführt wird.

2. Verfahren gemäß Anspruch 1,
**dadurch gekennzeichnet,**
**dass** der Ammoniak in Verfahrensschritt B) bezogen auf die Hydroxylgruppen im primären Alkohol in einem molaren Verhältnis von mindestens 5 zu 1, bevorzugt 50 zu 1, besonders bevorzugt 500 zu 1, eingesetzt wird.

3. Verfahren gemäß Anspruch 1 oder 2,
**dadurch gekennzeichnet,**
**dass** der primäre Alkohol eine Carboxyl- oder Estergruppe aufweist.

4. Verfahren gemäß Anspruch 1 oder 2,
**dadurch gekennzeichnet,**
**dass** der primäre Alkohol mindestens einen aliphatischen Alkylrest enthaltend mindestens drei kovalent miteinander verbundenen Kohlenstoffatome und kein quartäres Kohlenstoffatom aufweist.

5. Verfahren gemäß Anspruch 1 oder 2,
**dadurch gekennzeichnet,**
**dass** der primäre Alkohol keine Heteroatome aufweist.

6. Verfahren gemäß mindestens einem der vorherigen Ansprüche,
**dadurch gekennzeichnet,**
**dass** der aliphatische Alkylrest ein linearer oder verzweigter Alkylrest, enthaltend mindestens 4, bevorzugt mindestens 6, insbesondere mindestens 9 Kohlenstoffatome, ist.

7. Verfahren gemäß mindestens einem der vorherigen Ansprüche,
**dadurch gekennzeichnet,**
**dass** der primäre Alkohol ausgewählt ist aus 1-Butanol, 2-Methyl-1-propanol, 1-Pentanol, 3-Methyl-1-butanol, 2-Methyl-1-butanol, 1-Hexanol, 4-Methyl-1-pentanol, 3-Methyl-1-pentanol, 2-Methyl-1-pentanol, 2-Ethyl-1-butanol, Tripropylenglycol und die Anhydrohexitole, insbesondere Tripropylenglycol.

8. Verfahren gemäß mindestens einem der vorherigen Ansprüche,
**dadurch gekennzeichnet,**
**dass** der primäre Alkohol in einer Konzentration von zwischen 0,1 und 10000 mmol/L, bevorzugt zwischen 0,1 und 1000 mmol/L und besonders bevorzugt zwischen 0,1 und 100 mmol/L, eingesetzt wird.

9. Verfahren gemäß mindestens einem der vorherigen Ansprüche,
**dadurch gekennzeichnet,**
**dass** der in Verfahrensschritt B) eingesetzte Katalysator ausgewählt ist aus der Gruppe bestehend aus Alkalimetall-, Aluminium- und Lanthanid-Alkoxide, anorganische Verbindungen von Edelmetallen, mono- oder multimetallische, ein- oder mehrkernige Koordinationsverbindungen von einem oder mehreren Edelmetallen ausgewählt aus den Elementen Ruthenium, Rhodium, Iridium und Palladium sowie der anderen Platinmetalle und Eisen.

10. Verfahren gemäß mindestens einem der vorherigen Ansprüche,
**dadurch gekennzeichnet,**
**dass** in Verfahrensschritt B) flüssiger oder überkritischer Ammoniak und/oder eine Lösung von Ammoniumsalzen in einem Lösungsmittel eingesetzt werden.

11. Verfahren gemäß mindestens einem der vorherigen Ansprüche,
**dadurch gekennzeichnet,**
**dass** Verfahrensschritt B) bei einem Druck im Bereich zwischen 20 und 1000 bar, bevorzugt zwischen 20 und 500 bar und besonders bevorzugt zwischen 20 und 100 bar, durchgeführt wird.

12. Verfahren gemäß mindestens einem der vorherigen Ansprüche,
**dadurch gekennzeichnet,**
**dass** Verfahrensschritt B) in einem Temperaturbereich zwischen 80 und 220°°C, bevorzugt zwischen 90 und 200° C und besonders bevorzugt zwischen 100 und 170° C, durchgeführt wird.

13. Verfahren gemäß mindestens einem der vorherigen Ansprüche,
**dadurch gekennzeichnet,**
**dass** es in Abwesenheit von Wasserstoff durchgeführt wird.

## Claims

1. Process for preparing primary amines comprising the process steps
A) provision of a solution of a primary alcohol in a fluid, nongaseous phase,
B) contacting of the phase with free ammonia and/or at least one ammonia-releasing compound and a homogeneous catalyst and optionally
C) isolation of the primary amine formed in process step B),
**characterized**
**in that** the volume ratio of the volume of the liquid phase to the volume of the gas phase in process step B is greater than 0.05 and/or
**in that** process step B is carried out at pressures greater than 10 bar.

2. Process according to Claim 1,
**characterized**
**in that** the ammonia is preferably used in process step B) in a molar ratio based on the hydroxyl groups in the primary alcohol of at least 5:1, preferably 50:1, particularly preferably 500:1.

3. Process according to Claim 1 or 2,
**characterized**
**in that** the primary alcohol has a carboxyl or ester group.

4. Process according to Claim 1 or 2,
**characterized**
**in that** the primary alcohol has at least one aliphatic alkyl radical containing at least three carbon atoms covalently bound to one another and no quaternary carbon atom.

5. Process according to Claim 1 or 2,
**characterized**
**in that** the primary alcohol does not have any heteroatoms.

6. Process according to at least one of the preceding claims,
**characterized**
**in that** the aliphatic alkyl radical is a linear or branched alkyl radical containing at least 4, preferably at least 6, in particular at least 9, carbon atoms.

7. Process according to at least one of the preceding claims,
**characterized**
**in that** the primary alcohol is selected from among 1-butanol, 2-methyl-1-propanol, 1-pentanol, 3-methyl-1-butanol, 2-methyl-1-butanol, 1-hexanol, 4-methyl-1-pentanol, 3-methyl-1-pentanol, 2-ethyl-1-pentanol, 2-ethyl-1-butanol, tripropylene glycol and the anhydrohexitols, in particular tripropylene glycol.

8. Process according to at least one of the preceding claims,
**characterized**
**in that** the primary alcohol is used in a concentration of from 0.1 to 10000 mmol/l, preferably from 0.1 to 1000 mmol/l and particularly preferably from 0.1 to 100 mmol/l.

9. Process according to at least one of the preceding claims,
**characterized**
**in that** the catalyst used in process step B) is selected from the group consisting of alkali metal alkoxides, aluminium alkoxides and lanthanide alkoxides, inorganic compounds of noble metals, monometallic or multimetallic, mononuclear or multinuclear coordination compounds of one or more noble metals selected from among the elements ruthenium, rhodium, iridium and palladium and the other platinum metals and iron.

10. Process according to at least one of the preceding claims,
**characterized**
**in that** liquid or supercritical ammonia and/or a solution of ammonium salts in a solvent is/are used in process step B).

11. Process according to at least one of the preceding claims,
**characterized**
**in that** process step B) is carried out at a pressure in the range from 20 to 1000 bar, preferably from 20 to 500 bar and particularly preferably from 20 to 100 bar.

12. Process according to at least one of the preceding claims,
**characterized**
**in that** process step B) is carried out in a temperature range from 80 to 220°C, preferably from 90 to 200°C and particularly preferably from 100 to 170°C.

13. Process according to at least one of the preceding claims,
**characterized**
**in that** it is carried out in the absence of hydrogen.

## Revendications

1. Procédé pour la préparation d'amines primaires, comprenant les étapes de procédé, consistant à
A) mettre à disposition une solution d'un alcool primaire dans une phase fluide, non gazeuse,
B) mettre en contact la phase avec de l'ammoniac libre et/ou au moins un composé libérant de l'ammoniac et un catalyseur homogène et le cas échéant
C) isoler l'amine primaire formée dans l'étape de procédé B)
**caractérisé en ce que** le rapport volumique du volume de la phase liquide au volume de la phase gazeuse dans l'étape de procédé B est supérieur à 0,05 et/ou **en ce que** l'étape de procédé B est réalisée à des pressions supérieures à 10 bars.

2. Procédé selon la revendication 1, **caractérisé en ce que** l'ammoniac dans l'étape de procédé B) est utilisé, par rapport aux groupes hydroxyle dans l'alcool primaire, dans un rapport molaire d'au moins 5:1, de préférence de 50:1, de manière particulièrement préférée de 500:1.

3. Procédé selon la revendication 1 ou 2, **caractérisé en ce que** l'alcool primaire présente un groupe carboxyle ou ester.

4. Procédé selon la revendication 1 ou 2, **caractérisé en ce que** l'alcool primaire présente au moins un radical alkyle aliphatique contenant au moins trois atomes de carbone liés les uns aux autres par covalence et ne présente pas d'atome de carbone quaternaire.

5. Procédé selon la revendication 1 ou 2, **caractérisé en ce que** l'alcool primaire ne présente pas d'hétéroatomes.

6. Procédé selon au moins l'une quelconque des revendications précédentes, **caractérisé en ce que** le radical alkyle aliphatique est un radical alkyle linéaire ou ramifié, contenant au moins 4, de préférence au moins 6, en particulier au moins 9 atomes de carbone.

7. Procédé selon au moins l'une quelconque des revendications précédentes, **caractérisé en ce que** l'alcool primaire est choisi parmi le 1-butanol, le 2-méthyl-1-propanol, le 1-pentanol, le 3-méthyl-1-butanol, le 2-méthyl-1-butanol, le 1-hexanol, le 4-méthyl-1-pentanol, le 3-méthyl-1-pentanol, le 2-méthyl-1-pentanol, le 2-éthyl-1-butanol, le tripropylèneglycol et les anhydrohexitols, en particulier le tripropylèneglycol.

8. Procédé selon au moins l'une quelconque des revendications précédentes, **caractérisé en ce que** l'alcool primaire est utilisé en une concentration entre 0,1 et 10.000 mmoles/l, de préférence entre 0,1 et 1000 mmoles/L et de manière particulièrement préférée entre 0,1 et 100 mmoles/l.

9. Procédé selon au moins l'une quelconque des revendications précédentes, **caractérisé en ce que** le catalyseur utilisé dans l'étape de procédé B) est choisi dans le groupe constitué par les oxydes de métal alcalin, d'aluminium et de lanthanides, les composés inorganiques des métaux nobles, les composés de coordination monométalliques ou multimétalliques, à un ou plusieurs noyaux, d'un ou de plusieurs métaux nobles choisis parmi les éléments ruthénium, rhodium, iridium et palladium ainsi que les autres métaux du groupe du platine et le fer.

10. Procédé selon au moins l'une quelconque des revendications précédentes, **caractérisé en ce qu'**on utilise, dans l'étape de procédé B), de l'ammoniac liquide ou surcritique et/ou une solution de sels d'ammonium dans un solvant.

11. Procédé selon au moins l'une quelconque des revendications précédentes, **caractérisé en ce que** l'étape de procédé B) est réalisée à une pression dans la plage entre 20 et 1000 bars, de préférence entre 20 et 500 bars et de manière particulièrement préférée entre 20 et 100 bars.

12. Procédé selon au moins l'une quelconque des revendications précédentes, **caractérisé en ce que** l'étape de procédé B) est réalisée dans une plage de température entre 80 et 220°C, de préférence entre 90 et 200°C et de manière particulièrement préférée entre 100 et 170°C.

13. Procédé selon au moins l'une quelconque des revendications précédentes, **caractérisé en ce qu'**il est réalisé en l'absence d'hydrogène.
